(19) Europäisches Patentamt
European Patent Office
Office européen des brevets

(11) Veröffentlichungsnummer : **0 215 347 B1**

(12) # EUROPÄISCHE PATENTSCHRIFT

(45) Veröffentlichungstag der Patentschrift : **24.01.90**

(51) Int. Cl.$^5$ : **C 07 D291/06**

(21) Anmeldenummer : **86111840.4**

(22) Anmeldetag : **27.08.86**

(54) **Verfahren zur Herstellung von 6-Methyl-3,4-dihydro-1,2,3-oxathiazin-4-one-2,2-dioxid sowie zu dessen Reinigung.**

(30) Priorität : **03.09.85 DE 3531359**

(43) Veröffentlichungstag der Anmeldung : **25.03.87 Patentblatt 87/13**

(45) Bekanntmachung des Hinweises auf die Patenterteilung : **24.01.90 Patentblatt 90/04**

(84) Benannte Vertragsstaaten : **AT BE CH DE FR GB IT LI NL SE**

(56) Entgegenhaltungen :
**EP--A-- 0 155 634**
**DE--A-- 3 410 440**
**DE--B-- 2 327 804**
**ANGEWANDTE CHEMIE, Band 85, Nr. 22, 1973, Seiten 965-973; K. CLAUSS et al.: "Oxathiazinondioxide, eine neue Gruppe von Süssstoffen"**

(73) Patentinhaber : **HOECHST AKTIENGESELLSCHAFT**
**Postfach 80 03 20**
**D-6230 Frankfurt am Main 80 (DE)**

(72) Erfinder : **Reuschling, Dieter, Dr.**
**Beethovenstrasse 27**
**D-6308 Butzbach (DE)**
Erfinder : **Linkies, Adolf, Dr.**
**Loreleistrasse 12**
**D-6230 Frankfurt am Main 80 (DE)**
Erfinder : **Reimann, Walter, Dr.**
**Thüringer Weg 33**
**D-6238 Hofheim am Taunus (DE)**
Erfinder : **Schweikert, Otto Ernst, Dr.**
**Freiherr-vom-Stein-Strasse 37**
**D-6233 Kelkheim (Taunus) (DE)**
Erfinder : **Mack, Karl Ernst, Dr.**
**Klingenbachstrasse 43**
**D-6200 Wiesbaden (DE)**

**EP 0 215 347 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

6-Methyl-3,4-dihydro-1,2,3-oxathiazin-4-on,-2,2-dioxid ist die Verbindung der Formel

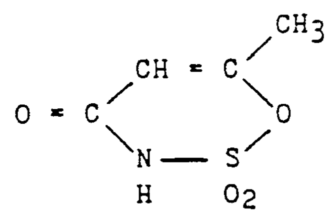

Infolge des aciden Wasserstoffs am Stickstoffatom ist die Verbindung zur Salzbildung (mit Basen) befähigt. Die nicht-toxischen Salze — wie z. B. das Na-, das K- und das Ca-Salz — können wegen ihres z. T. intensiven Süßgeschmacks als Süßstoffe auf dem Nahrungsmittelsektor verwendet werden, wobei das K-Salz (« Acesulfam K » oder auch nur « Acesulfam ») von besonderer Bedeutung ist.

Zur Herstellung des 6-Methyl-3,4-dihydro-1,2,3-oxathiazin-4-on-2,2-dioxids und dessen nicth-toxischer Salze ist eine Reihe verschiedener Verfahren bekannt ; vgl. Angewandte Chemie 85, Heft 22 (1973) S. 965 bis 73, entsprechend International Edition Vol. 12, No. 11 (1973), S. 869-76. Praktisch alle Verfahren gehen von Chlor- oder Fluorsulfonylisocyanat ($XSO_2NCO$ mit X = Cl oder F) aus. Das Chlor- bzw. Fluorsulfonylisocyanat wird dann mit Monomethylacetylen, Aceton, Acetessigsäure, Acetessigsäuretert.-butylester oder Benzylpropenylether (in einer meist mehrstufigen Reaktion) zu Acetoacetamid-N-sulfochlorid bzw. -fluorid umgesetzt, was unter der Einwirkung von Basen (wie z. B. methanolischer KOH) cyclisiert und die entsprechenden Salze des 6-Methyl-3,4-dihydro-1,2,3-oxa-thiazin-4-on-2,2-dioxids liefert. Aus den Salzen kann das freie Oxathiazinon gewünschtenfalls auf übliche Weise (mit Säuren) erhalten werden.

Ein weiteres Verfahren zur Herstellung der Oxathiazinon-Zwischenstufe Acetoacetamid-N-sulfofluorid geht aus von Amidosulfofluorid $H_2NSO_2F$, dem partiellen Hydrolyseprodukt des Fluorsulfonylisocyanats (DE-OS 2 453 063). Danach wird das Fluorid der Amidosulfonsäure $H_2NSO_2F$ mit einer etwa äquimolaren Menge des Acetoacetylierungsmittels Diketen in einem inerten organischen Lösungsmittel in Gegenwart eines Amins bei Temperaturen zwischen etwa — 30 und 100 °C umgesetzt ; die Umsetzung verläuft nach folgender Reaktionsgleichung (mit Triethylamin als Amin) :

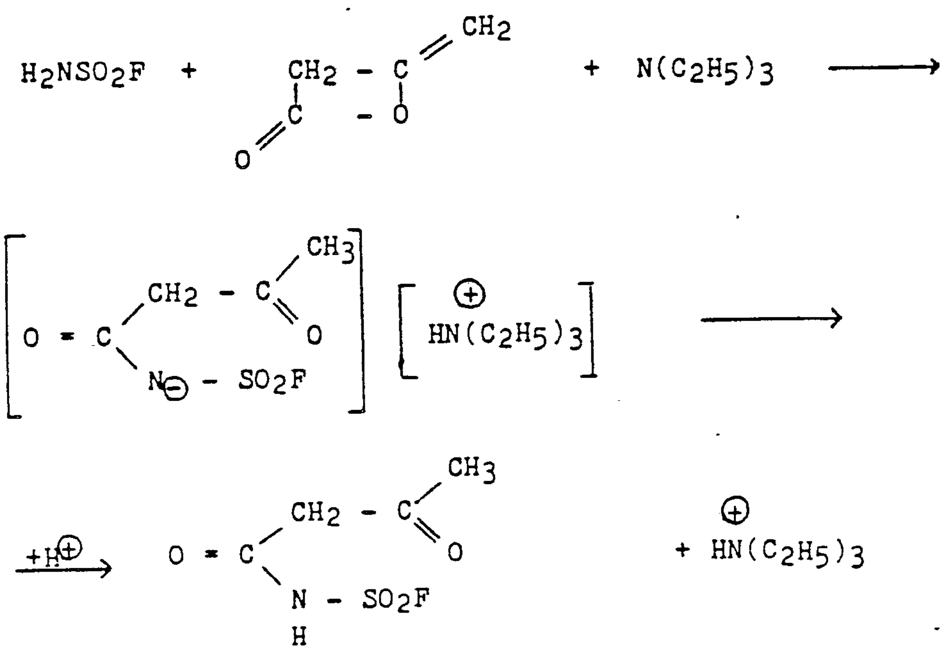

Acetoacetamid-N-sulfofluorid

Das Acetoacetamid-N-sulfofluorid wird dann auf übliche Weise mittels einer Base, z. B. mit methanolischer KOH, zum Süßstoff cyclisiert :

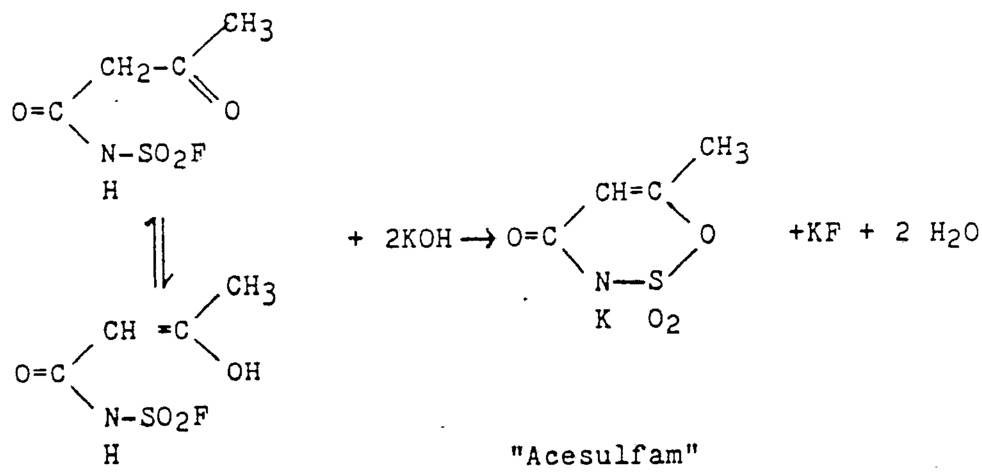


"Acesulfam"

Obwohl die bekannten Verfahren z. T. recht befriedigende Ausbeuten an 6-Methyl-3,4-dihydro-1,2,3-oxathiazin-4-on-2,2-dioxid und dessen nicht-toxischen Salzen liefern, (bis zu ca. 85 % d. Th., bezogen auf das Ausgangs-Amidosulfonsäurehalogenid), sind sie wegen der Notwendigkeit des Einsatzes der nicht ganz einfach zugänglichen Ausgangsstoffe Chlor- bzw. Fluorsulfonylisocyanat vor allem für technische Belange noch verbesserungsbedürftig ; die Herstellung des Chlor- und Fluor-sulfonylisocyanats erfordert nämlich wegen der z. T. ziemlich unangenehm handzuhabenden Ausgangsmaterialien (HCN, $CL_2$, $SO_3$ und HF) erhebliche Vorsichtsmaßnahmen und Sicherheitsvorkehrungen. Der Herstellung des Chlor- und Fluor-sulfonylisocyanats liegen folgende Reaktionsgleichungen zugrunde :

$$HCN + Cl_2 \rightarrow ClCN + HCl$$
$$ClCN + SO_3 \rightarrow ClSO_2NCO$$
$$ClSO_2NCO + HF \rightarrow FSO_2NCO + HCl$$

Der Ersatz des Amidosulfofluorids in dem Verfahren gemäß der vorerwähnten DE-OS 24 53 063 etwa durch die wesentlich leichter (z. B. aus $NH_3 + SO_3$) erhältliche Amidosulfonsäure $H_2NSO_3H$ bzw. deren Salze erschien kaum erfolgversprechen, weil nämlich die Umsetzung des Na-Amidosulfonats $H_2NSO_3Na$ mit Diketen in wässrig-alkalischer Lösung überhaupt kein rein isolierbares Umsetzungsprodukt ergibt. Das bei dieser Umsetzung wohl zumindest mit entstandene 1 : 1-Addukt konnte vielmehr nur in Form des Kupplungsproduktes mit 4-Nitrophenyldiazoniumchlorid als blaßgelber Farbstoff gewonnen werden ; vgl. Ber. 83 (1950), S. 551-558, insbesondere S. 555, letzter Absatz vor der Beschreibung der Versuche und S. 558, letzter Absatz :

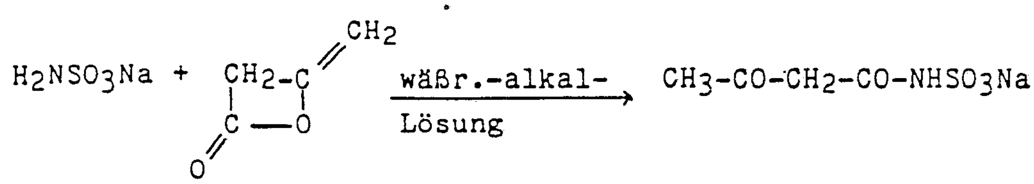


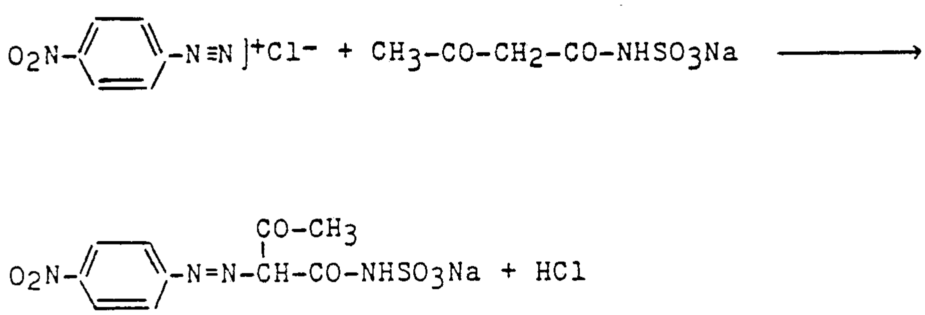

Die Acetoacetamid-N-sulfonsäure ist im übrigen ansonsten nur bzw. auch als Zwischenprodukt bei der Zersetzung des 6-Methyl-3,4-dihydro-1,2,3-oxathiazin-4-on-2,2-dioxids während des Kochens in wässriger Lösung postuliert worden ; vgl. die anfangs zitierte Literatur Angew. Chemie (1973) a.a.O. :

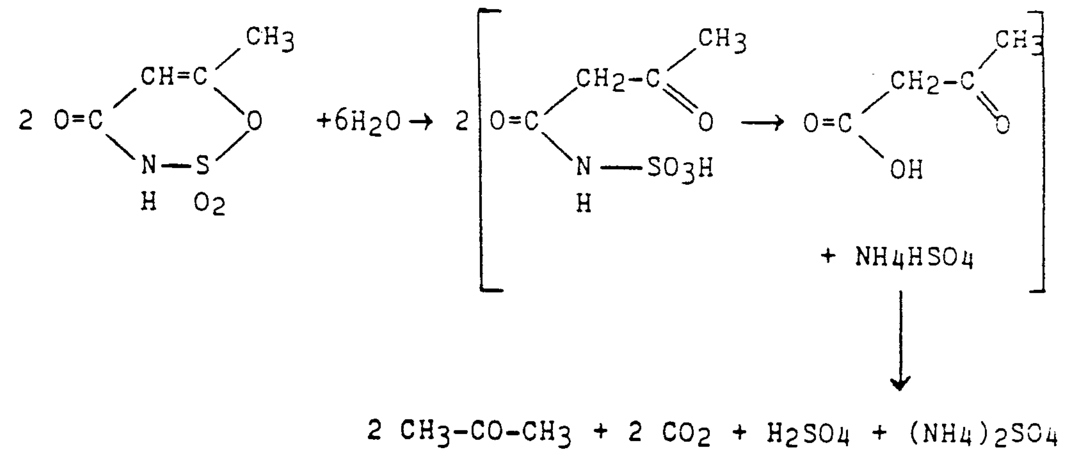

Wegen der insbesondere infolge der Notwendigkeit des Einsatzes nicht ganz einfach zugänglicher Ausgangsstoffe vor allem für die Durchführung in technischem Maßstab nicht ganz befriedigenden Verfahren des Standes der Technik zur Herstellung von 6-Methyl-3,4-dihydro-1,2,3-oxathiazin-4-on-2,2-dioxid und dessen nicht-toxischer Salze bestand somit die Aufgabe, die bekannten Verfahren entsprechend zu verbessern oder ein neues verbessertes Verfahren zu entwickeln.

Zur Lösung dieser Aufgabe wurde bereits vorgeschlagen das Verfahrens gemäß DE-OS 2 453 063 hauptsächlich in der Weise zu modifizieren, daß man das Amidosulfofluorid in dem bekannten Verfahren durch Salze der Amidosulfonsäure ersetzt und das erhaltene Acetoacetylierungsprodukt nachfolgend mittels $SO_3$ zum Ring schließt (EP-Patentanmeldung Nr. 85 102 885.2 — Veröffentlichung EP-A 0155634 — mit Priorität der deutschen Anmeldung P 34 10 439.9 vom 22.3.1984).

Die zuletzt genannte Patentanmeldung bezieht sich speziell auf ein Verfahren zur Herstellung von 6-Methyl-3,4-dihydro-1,2,3-oxathiazin-4-on-2,2-dioxid und dessen nichttoxischen Salzen durch

a) Umsetzung eines Amidosulfonsäurederivates mit einer mindestens etwa äquimolaren Menge eines Acetoacetylierungsmittels in einem inertem organischen Lösungsmittel, ggf. in Gegenwart eines Amin- oder Phosphin-Katalysators, zu einem Acetoacetamidderivat und

b) Ringschluß des Acetoacetamidderivats ;

das Verfahren ist dadurch gekennzeichnet, daß man in Stufe a) als Amidosulfonsäurederivat ein in dem eingesetzten inerten organischen Lösungsmittel zumindest teilweise lösliches Salz der Amidosulfonsäure verwendet, daß man das is dieser Stufe gebildete Acetoacetamid-N-sulfonat oder auch die freie Acetoacetamid-N-sulfonsäure in Stufe b) durch die Einwirkung der mindestens äquimolaren Menge von $SO_3$, gegebenenfalls in einem inerten anorganischen oder organischen Lösungsmittel, zum Ring des 6-Methyl-3,4-dihydro-1,2,3-oxathiazin-4-on-2,2-dioxids schließt und daß man das hier in der Säureform anfallende Produkt dann gegebenenfalls noch in einer Stufe c) mit einer Base neutralisiert.

Als dem Verfahren zugrundeliegenden Reaktionsgleichungen sind in der vorerwähnten Patentanmeldung angegeben (mit Diketen als Acetoacetylierungsmittel)

(Siehe Scheme Seite 5 f.)

a)

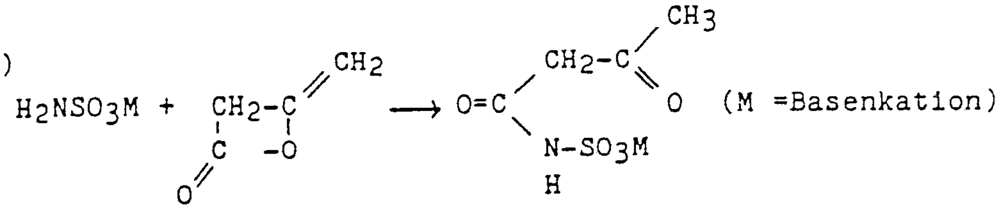


b)

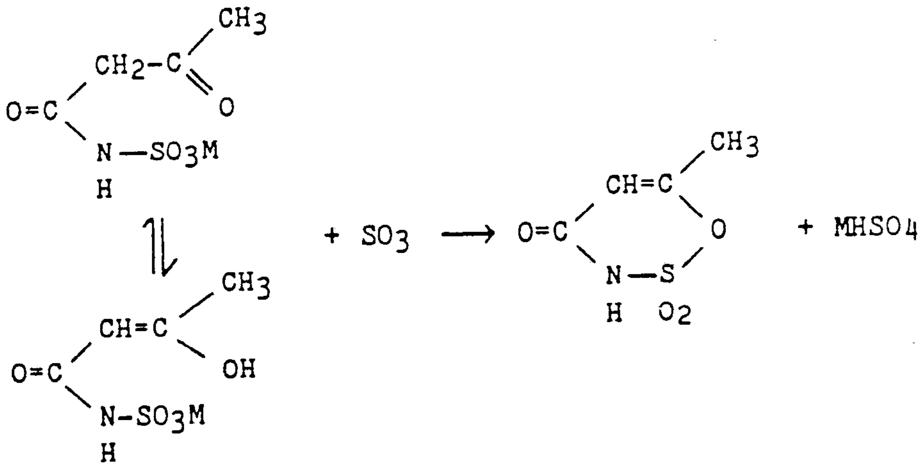

c)

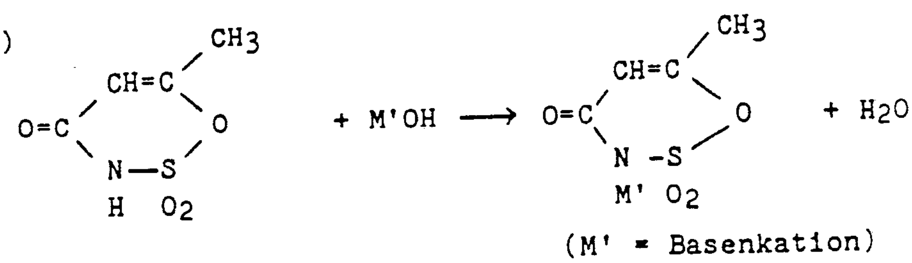


In diesem Reaktionsschema ist Stufe b) mit einer gegenüber dem Acetoacetamid-N-Sulfonat äquimolaren $SO_3$-Menge dargestellt. Bevorzugt wird jedoch ein $SO_3$-Überschuß verwendet. Dabei entsteht ein in seiner chemischen Struktur noch nicht genau bekanntes Zwischenprodukt, das jedoch möglicherweise ein $SO_3$-Addukt des 6-Methyl-3,4-dihydro-1,2,3-oxathiazin-4-on-2,2-dioxids — nachfolgend als « $SO_3$-Addukt » bezeichnet — darstellt, welches dann noch hydrolysiert werden muß. In diesem Falle besteht die vorerwähnte Reaktionsstufe b) also aus 2 Teilstufen, nämlich

b1 : Ringschluß

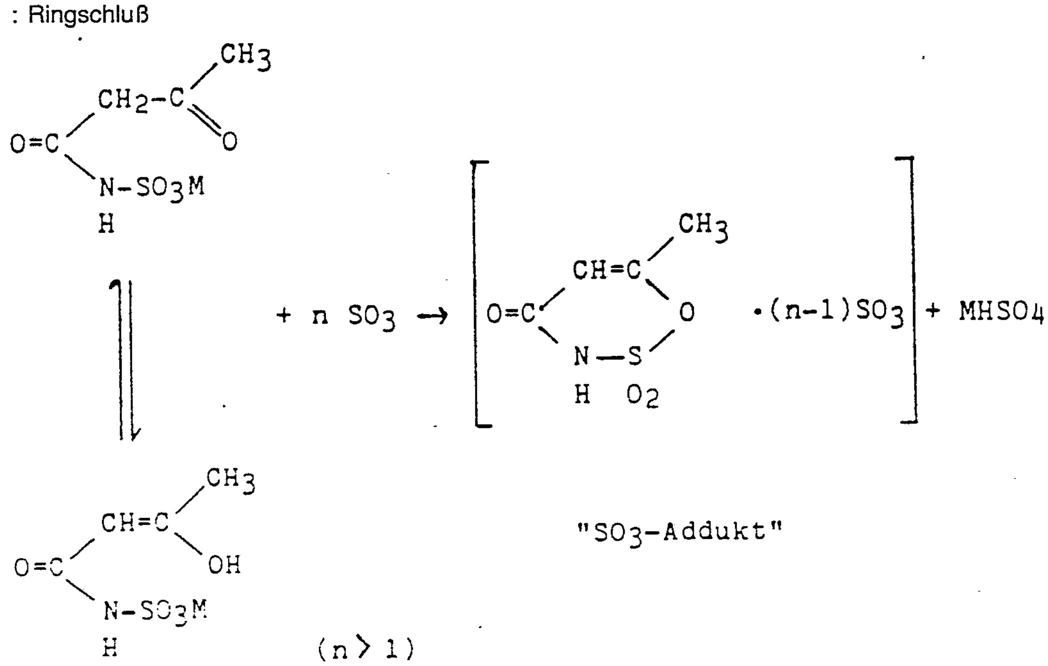

b2: Hydrolyse

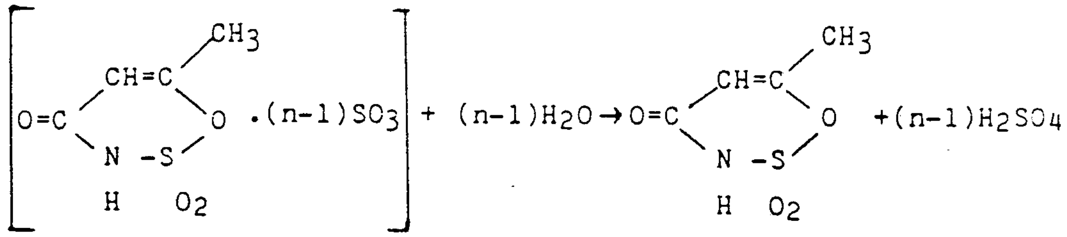

Die Ringschlußreaktion (b1) wird nach der vorerwähnten Patentanmeldung bei Temperaturen zwischen etwa — 70 und + 175 °C, vorzugsweise zwischen etwa — 40 und + 10 °C, durchgeführt ; die Reaktionszeiten liegen zwischen etwa 1 und 10 Stunden.

Die Hydrolyse (b2) erfolgt nach der Ringschlußreaktion durch Zugabe von Wasser oder Eis.

Die Aufarbeitung geschieht dann auf übliche Weise ; näher wird die Aufarbeitung jedoch nur für den bevorzugten Fall der Verwendung von Methylenchlorid als Reaktionsmedium erläutert. In diesem Fall bilden sich nach der Hydrolyse 2 Phasen, wobei sich das 6-Methyl-3,4-dihydro-1,2,3-oxathiazin-4-on-2,2-dioxid hauptsächlich in die organische Phase begibt. Die noch in der wässrigen Schwefelsäure befindlichen Anteile können durch Extraktion mit einem (mit Wasser nicht mischbaren) organischen Lösungsmittel wie z. B. mit Methylenchlorid oder einem organischen Ester gewonnen werden.

Oder man destilliert nach der Zugabe von Wasser das Raktionslösemittel ab und extrahiert das in der Reaktionsschwefelsäure zurückbleibende 6-Methyl-3,4-dihydro-1,2,3-oxathiazin-4-on-2,2-dioxid mit einem besser geeigneten organischen Lösemittel.

Die vereinigten organischen Phasen werden z. B. mit $Na_2SO_4$ getrocknet und eingeengt. Bei der Extraktion gegebenenfalls mitgerissene Schwefelsäure kann man durch gezielte Zugabe wässriger Lauge zur organischen Phase entfernen. Falls die Gewinnung des 6-Methyl-3,4-dihydro-1,2,3-oxathiazin-4-on-2,2-dioxids beabsichtigt ist, wird es zweckmäßig noch auf übliche Weise gereinigt (vorzugsweise durch Umkristallisation). Die Ausbeute liegt zwischen etwa 70 und 95 % d. Th., bezogen auf das Acetoactamid-N-sulfonat (bzw. die freie Säure).

Wenn jedoch die Gewinnung eines nicht-toxischen Salzes des 6-Methyl-3,4-dihydro-1,2,3-oxathiazin-4-on-2,2-dioxids beabsichtigt ist, folgt noch die Neutralisationsstufe c). Dazu wird die in Stufe b) in der Säureform anfallende Oxathiazinon-Verbindung auf übliche Weise mit einer entsprechenden Base neutralisiert. Zu diesem Zweck werden beispielsweise die am Ende der Stufe b) vereinigten, getrockneten und eingeengten organischen Phasen in geeigneten organischen Lösungsmitteln, wie z. B. Alkoholen, Ketonen, Estern oder Ethern oder auch in Wasser mit einer entsprechenden Base — vorzugsweise mit einer Kaliumbase wie z. B. KOH, $KHCO_3$, $K_2CO_3$, K-Alkoholate etc. — neutralisiert. Oder die Oxathiazinon-Verbindung wird aus der gereinigten organischen Extraktionsphase (Stufe b) mit wässriger Kaliumbase direkt extraktiv neutralisiert. Das Oxathiazinon-Salz fällt dann, gegebenenfalls nach Einengen der Lösung, in kristalliner Form aus und kann zur Reinigung noch umkristallisiert werden. Die Neutralisationsstufe verläuft mit praktisch 100 %iger Ausbeute.

Hinsichtlich der weiteren Verfahrensdetails wird auf die ausführliche Beschreibung in der genannten Patentanmeldung verwiesen.

Das Verfahren geht von einfach zugänglichen und wohlfeilen Ausgangsstoffen aus und ist außerordentlich einfach durchführbar. Die Ausbeuten des Gesamtverfahrens liegen zwischen etwa 65 und 95 % d. Th., bezogen auf das Ausgangs-Amidosulfonat.

Im Zuge der weiteren Bearbeitung dieses Verfahrens wurde auch vorgeschlagen, sowohl die Ringschlußreaktion (b1) als auch die Hydrolyse (b2) in kurzen bis sehr kurzen Zeiten (ca. 10 Minuten bis herab in den Bereich von Sekunden und Sekundenbruchteilen) durchzuführen (Patentanmeldung P 35 27 070.5 vom 29.7.1985 — DE-A-3527070). Die praktische Ausführung geschieht vorzugsweise in Vorrichtungen, welche für die Durchführung derartiger schnell und unter Wärmeentwicklung ablaufender Reaktionen geeignet und bekannt sind (Dünnschicht-, Fallfilm-, Sprühreaktoren, Rohrreaktoren mit und ohne Einbauten, etc.). Die Aufarbeitung des Reaktionsgemisches erfolgt wie in der zuvor genannten Patentanmeldung beschrieben. Durch diese « Kurzzeitvariante » läßt sich die technische Durchführung und insbesondere die Raum-Zeit-Ausbeute des Verfahrens erheblich verbessern.

Schließlich wurde auch schon vorgeschlagen, anstelle der Stufen a) und b) des Verfahrens der vorerwähnten EP-A-0155634 Acetoacetamid mit der mindestens etwa 2-molaren Menge $SO_3$, gegebenenfalls in einem inerten anorganischen oder organischen Lösungsmittel, umzusetzen (DE-Patentanmeldung P 34 10 440.2 vom 22.3.1984 — DE-A 3410440. Hierbei entsteht in einem Schritt wahrscheinlich zuerst aus einem Mol Acetoacetamid und einem Mol $SO_3$ Acetoacetamid-N-sulfonsäure, die dann mit einem weiteren Mol $SO_3$ zum 6-Methyl-3.4-dihydro-1,2,3-oxathiazin-4-on-2,2-dioxid cyclisiert entsprechend dem folgenden Reaktionsschema :

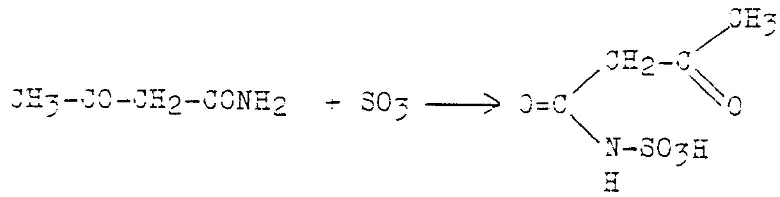

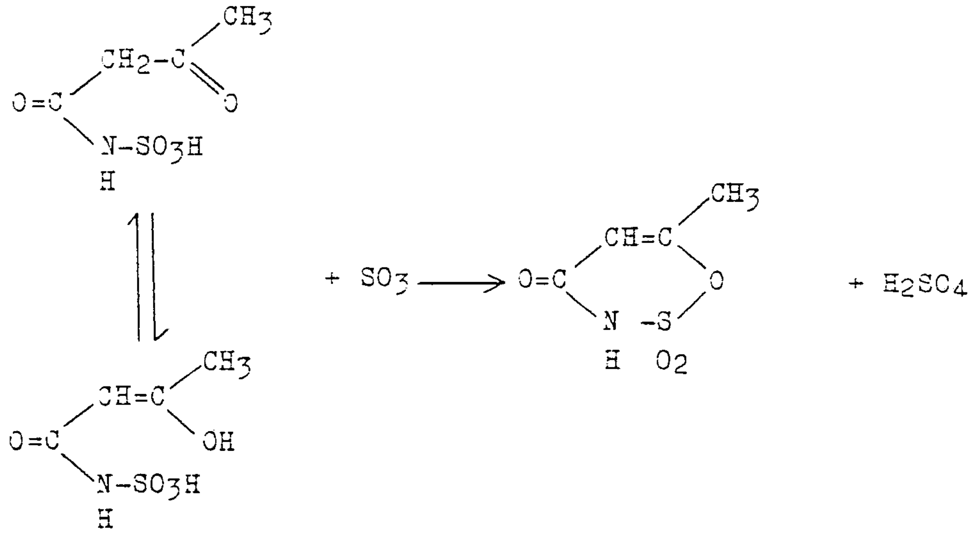

Mit überschüssigem $SO_3$ entsteht auch hier das « $SO_3$-Addukt », das zwecks Freisetzung des 6-Methyl-3,4-dihydro-1,2,3-oxathiazin-4-on-2,2-dioxids noch hydrolysiert werden muß. Die Aufarbeitung des hydrolysierten Ansatzes sowie gegebenenfalls die Überführung des 6-Methyl-3,4-dihydro-1,2,3-oxathiazin-4-on-2,2-dioxids in dessen nicht-toxische Salze geschieht im Prinzip in der gleichen Weise wie dies in der vorerwähnten EP-A 0155634 beschrieben ist. Die Ausbeuteangaben für das 6-Methyl-3,4-dihydro-1,2,3-oxathiazin-4-on-2,2-dioxid liegen zwischen etwa 30 und 90 % d. Th., bezogen auf das Ausgangs-Acetoacetamid.

Nach allen drei vorerwähnten Patentanmeldungen wird das bei der Hydrolyse des « $SO_3$-Addukts » in Freiheit gesetzte 6-Methyl-3,4-dihydro-1,2,3-oxathiazin-4-on-2,2-dioxid aus der organischen Phase gewonnen, welche sich bei Verwendung eines (mit Wasser nicht mischbaren) organischen Reaktionslösemittels nach dem Wasserzusatz bildet und/oder welche bei der Extraktion der Reaktionsschwefelsäure mit organischen Lösemitteln entsteht. Das so gewonnene 6-Methyl-3,4-dihydro-1,2,3-oxathiazin-4-on-2,2-dioxid sowie auch die daraus gegebenenfalls durch Umsetzung mit entsprechenden Basen erhaltenen nicht-toxischen Salze sind jedoch nicht immer von der erforderlichen Reinheit, so daß oft noch verschiedene — mit zusätzlichem Aufwand sowie auch mit Substanzverlusten verbundene — Reinigungsoperationen — vorzugsweise Umkristallisation(en) — notwendig sind.

In weiterer Ausbildung der vorerwähnten Verfahren wurde nun gefunden, daß man ein erheblich reineres 6-Methyl-3,4-dihydro-1,2,3-oxathiazin-4-on-2,2-dioxid erhält, wenn man es nicht aus der — wie vorstehend beschrieben — organischen Phase, sondern aus der wässrig-schwefelsauren Phase direkt durch Kristallisation gewinnt.

Erfindungsgegenstand ist ein Verfahren zur Herstellung von 6-Methyl-3,4-dihydro-1,2,3-oxathiazin-4-on-2,2-dioxid durch Ringschluß eines Acetoacetamidderivats ; das Verfahren ist dadurch gekennzeichnet, daß man als Acetoacetamidderivat Acetoacetamid-N-sulfonsäure oder deren Salze — gelöst in einem mit Wasser nicht mischbaren inerten organischen Lösungsmittel — verwendet, daß man den Ringschluß durch die Einwirkung der mindestens äquimolaren Menge $SO_3$ — gegebenenfalls in gleicher Weise gelöst in einem mit Wasser nicht mischbaren inerten organischen Lösungsmittel oder auch in einem inerten anorganischen Lösungsmittel — durchführt, daß man im Falle des Einsatzes einer äquimolaren Menge $SO_3$ nach beendeter Ringschlußreaktion 20 bis 90 %ige wässrige Schwefelsäure zusetzt bzw. — im Falle des Einsatzes einer mehr als äquimolaren Menge $SO_3$ — das nach der Ringschlußreaktion als $SO_3$-Addukt anfallende 6-Methyl-3,4-dihydro-1,2,3-oxathiazin-4-on-2,2-dioxid hydrolysiert und daß man aus dem resultierenden Mehrphasengemisch das inerte organische Lösungsmittel abdestilliert und aus der verbleibenden wässrig-schwefelsauren Phase das 6-Methyl-3,4-dihydro-1,2,3-oxathiazin-4-on-2,2-dioxid durch Kristallisation gewinnt.

Das glatte Gelingen des Ringschlusses der Acetoacetamid-N-sulfonsäure und von deren Salzen mit $SO_3$ ist sehr überraschend, weil die unter Ringschluß erfolgende Wasser- bzw. Basen-Abspaltung nämlich mit anderen Wasser- bzw. Basen-abspaltenden Mitteln wie z. B. $P_2O_5$, Acetanhydrid, Trifluoressigsäureanhydrid, Thionylchlorid etc. nicht oder jedenfalls praktisch nicht gelingt, wie bereits in der vorerwähnten EP-A-0155634 anhand eines Vergleichsbeispiels (mit $P_2O_5$) gezeigt werden konnte.

Außerdem ist überraschend, daß bei der Kristallisation des 6-Methyl-3,4-dihydro-1,2,3-oxathiazin-4-on-2,2-dioxids aus Schwefelsäure ein Produkt anfällt, welches außer geringen Mengen anhaftender Schwefelsäure (die aber leicht entfernt werden kann) praktisch keine Verunreinigungen — jedenfalls praktisch keine Verunreinigungen organischer Natur — enthält, weil man durchaus hätte erwarten können, daß etwaige — von der vorhergehenden Reaktion her vorhandene — gelöste organische Verunreinigungen zusammen mit dem 6-Methyl-3,4-dihydro-1,2,3-oxathiazin-4-on-2,2-dioxid auskristallisieren.

Die Herstellung der Ausgangs-Acetacetamid-N-sulfonsäure und von deren Salzen erfolgt bevorzugt nach Stufe a) des Verfahrens der vorerwähnten EP-A-0155634 durch Umsetzung der Li- oder Ammonium-Salze der Amidosulfonsäure mit Diketen in inerten organischen Lösungsmitteln. Hierbei werden Lösungen der Li- und Ammonium-Salze der Acetoacetamid-N-sulfonsäure erhalten, welche als solche direkt für die Ringschlußreaktion mit $SO_3$ eingesetzt werden können.

Für die genannte Ringschlußreaktion können natürlich auch andere Salze der Acetoacetamid-N-sulfonsäure — insbesondere Alkali — und Erdalkalisalze — verwendet werden. Der Einsatz der freien Acetoacetamid-N-sulfonsäure bringt gegenüber den Salzen kaum Vorteile.

Wie die Salze, so kann auch die freie Acetoacetamid-N-sulfonsäure gleich in der entsprechenden Lösung wie sie bei der Herstellung anfällt, für die Ringschlußreaktion eingesetzt werden. Als Lösung wie sie bei der Herstellung anfällt, kann auch die Lösung der beim Verfahren der DE-Patentanmeldung DE-A-34 10 440 wohl intermediär gebildeten freien Acetoacetamid-N-sulfonsäure betrachtet werden.

Als inerte organische Lösemittel für die Acetoacetamid-N-sulfonsäure oder für deren Salze kommen zweckmäßig aus der Reihe der in den vorerwähnten Patentanmeldungen aufgeführten inerten organischen Lösemitteln diejenigen infrage, welche mit Wasser nicht mischbar sind und welche (bei Normaldruck) unter 100 °C sieden; d. s. halogenierte aliphatische Kohlenwasserstoffe, vorzugsweise mit bis zu 4 C-Atomen wie z. B. Methylenchlorid, Chloroform, 1,2-Dichlorethan, Trichlorethylen, Trichlorfluorethylen etc., sowie Kohlensäureester mit niederen aliphatischen Alkoholen, vorzugsweise mit Methanol.

Die organischen Lösemittel können sowohl einzeln als auch in Mischung eingesetzt werden.

Besonders bevorzugte Lösemittel sind halogenierte aliphatische Kohlenwasserstoffe, insbesondere Methylenchlorid.

Die Konzentration der Acetoacetamid-N-sulfonsäure oder von deren Salzen im inerten Lösemittel ist nicht kritisch, wird aber einerseits begrenzt durch die Löslichkeit, andererseits durch Wirtschaftlichkeitsüberlegungen, da bei hoher Verdünnung viel Lösemittel nachher wieder abgetrennt und aufgearbeitet werden muß. Im allgemeinen sind Konzentrationen zwischen etwa 0,1 und 2 Mol Acetoacetamid-N-sulfonsäure oder von deren Salzen pro Liter zweckmäßig.

Das $SO_3$ kann sowohl in fester oder flüssiger Form als auch durch Einkondensation von $SO_3$-Dampf zugegeben werden. Bevorzugt ist jedoch der Zusatzt in gelöster Form, und zwar gelöst in einem mit Wasser nicht mischbaren inerten organischen Lösemittel oder auch in einem inerten anorganischen Lösemittel.

Als mit Wasser nicht mischbare inerte organische Lösemittel kommen im Prinzip die gleichen in Frage, welche auch für die Lösung der Acetoacetamid-N-sulfonsäure oder von deren Salzen verwendet werden.

Als inerte anorganische Lösemittel können beispielsweise konzentrierte Schwefelsäure oder flüssiges $SO_2$ eingesetzt werden. Auch die Menge des für das $SO_3$ eingesetzten inerten Lösemittels ist im Prinzip nicht kritisch. Wenn ein Lösemittel eingesetzt wird, soll lediglich eine ausreichende Lösung des $SO_3$ gewährleistet sein; nach oben ist die Menge des Lösemittels von Wirtschaftlichkeitserwägungen begrenzt. Günstige Konzentrationen liegen bei etwa 5 bis 50 Gew.-%, vorzugsweise etwa 15 bis 30 Gew.-% $SO_3$.

In einer bevorzugten Ausführungsform der Erfindung wird sowohl für die Acetoacetamid-N-sulfonsäure bzw. deren Salze als auch für das $SO_3$ das gleiche inerte Lösemittel, vorzugsweise aus der Gruppe der halogenierten aliphatischen Kohlenwasserstoffe, insbesondere nur Methylenchlorid, verwendet.

Das Molverhältnis von Acetoacetamid-N-sulfonsäure bzw. -sulfonat : $SO_3$ kann zwar etwa 1 : 1 sein, bevorzugt ist aber ein bis zu etwa 20-facher $SO_3$-Überschuß, vorzugsweise ein etwa 3- bis 10-facher, insbesondere etwa 4- bis 7-facher molarer Überschuß.

Die Durchführung der Ringschlußreaktion erfolgt ansonsten im Prinzip in der gleichen Weise und unter den gleichen Bedingungen wie dies in den vorerwähnten 3 Patentanmeldungen beschrieben ist.

Wenn die Acetoacetamid-N-sulfonsäure oder deren Salze und das $SO_3$ in äquimolarer Menge eingesetzt werden, entsteht — wie aus den anfangs wiedergegebenen Reaktionsschemata ersichtlich ist — kein « $SO_3$-Addukt ». Eine Hydrolyse ist daher in diesem Fall nicht erforderlich. Für die Gewinnung von reinem 6-Methyl-3,4-dihydro-1,2,3-oxathiazin-4-on-2,2-dioxid wird hier Schwefelsäure vorzugsweiser einer Konzentration zwischen 20 und 90 %, insbesondere zwischen 50 und 85 %, zugesetzt. Deren Menge soll so bemessen werden, daß eine gute Kristallisation möglich ist, ohne daß zuviel Produkt gelöst bleibt; durch wenige einfache Handversuche ist die jeweils günstige Menge leicht zu ermitteln.

Im bevorzugten Fall des Einsatzes von Acetoacetamid-N-sulfonsäure oder von deren Salzen und $SO_3$ im Molverhältnis 1 : mehr als 1 entsteht bei der Ringschlußreaktion ein « $SO_3$-Addukt », aus dem das 6-Methyl-3.4-dihydro-1,2,3-oxathiazin-4-on-2,2-dioxid durch Hydrolyse freigesetzt werden muß. Die Hydrolyse erfolgt durch Zugabe von Wasser oder Eis, zweckmäßig in einer — im Verhältnis zum angewandten $SO_3$-Überschuß — etwa 2 — bis 6-fachen molaren Menge.

Nach dem Zusatz der Schwefelsäure nach der Ringschlußreaktion mit Acetoacetamid-N-sulfonsäure oder -sulfonat und $SO_3$ im Molverhältnis 1 : 1 sowie nach der Hydrolyse im Anschluß an die Ringschlußreaktion mit Acetoacetamid-N-sulfonsäure oder -sulfonat im Molverhältnis 1 : mehr als 1 liegt ein 2- oder (wenn bereits 6-Methyl-3,4-dihydro-1,2,3-oxathiazin-4-on-2,2-dioxid ausgefallen ist) 3-Phasengemisch vor. Das 6-Methyl-3,4-dihydro-1,2,3-oxathiazin-4-on-2,2-dioxid befindet sich im wesentlichen gelöst in der organischen und in der Schwefelsäurephase. Falls das inerte organische Lösemittel etwa durch Verdampfung gemäß der « Kurzzeitvariante » nach Patentanmeldung DE-A 35 27 070 bereits entfernt ist, befindet sich das 6-Methyl-3,4-dihydro-1,2,3-oxathiazin-4-on-2,2-dioxid hauptsächlich gelöst nur in der Schwefelsäurephase.

Wenn jedoch noch eine organische Phase vorhanden ist, wird das inerte organische Lösungsmittel aus dem gesamten Mehrphasengemisch abdestilliert. Die Destillation kann unter Vakuum, druchlos oder bei Überdruck erfolgen. Wird beispielsweise das als Lösemittel besonders bevorzugte Methylenchlorid verwendet, so erfolgt die Destillation vorteilhaft bei Drucken von etwa 200 mbar bis 1 bar und Temperaturen von etwa 0 bis 42 °C.

Man kann die Destillation sowohl diskontinuierlich als auch kontinuierlich vornehmen. In diskontinuierlicher Verfahrensweise kann das Lösemittel direkt aus dem Reaktionsgefäß abdestilliert werden. Bevorzugt ist jedoch die kontinuierliche Abdestillation bei kurzen Verweilzeiten, weil dadurch eine etwaige thermische Zersetzung des 6-Methyl-3,4-dihydro-1,2,3-oxathiazin-4-on-2,2-dioxids am besten vermieden wird. Als Destillat wird das zur Ringschlußreaktion verwendete inerte organische Lösemittel in sauberer Form erhalten.

Die nach der Destillation verbleibende Schwefelsäurephase enthält alles 6-Methyl-3,4-dihydro-1,2,3-oxathiazin-4-on-2,2-dioxid, aber auch alle Nebenprodukte und Verunreinigungen. Wenn die Abdestillation des organischen Lösemittels bei erhöhten Temperaturen vorgenommen wurde, ist auch die Schwefelsäurephase von erhöhter Temperatur. Beim Abkühlen kristallisiert dann das 6-Methyl-3,4-dihydro-1,2,3-oxathiazin-4-on-2,2-dioxid in überraschend hoher Reinheit — insbesondere frei von organischen Verunreinigungen — aus.

Wenn das organische Lösemittel bei niedrigerer Temperatur abdestilliert wurde kann die Schwefelsäurephase bis zur Erstarrung gegebenenfalls nicht mehr allzu sehr abgekühlt werden. In diesem Fall kann es zweckmäßig sein, die Schwefelsäurephase möglichst unter vermindertem Druck einzuengen, wodurch dann die Kristalle des 6-Methyl-3,4-dihydro-1,2,3-oxathiazin-4-on-2,2-dioxids ausfallen.

Falls bereits am Ende der Ringschlußreaktion und/oder beim Abdestillieren des organischen Lösemittels ein Niederschlag entsteht, kann es weiterhin zweckmäßig sein, diesen durch Erwärmen der Schwefelsäurephase oder gegebenenfalls auch durch Zusatz weiterer Schwefelsäure zur Auflösung zu bringen und die Kristallisation dann durch Abkühlen zu bewirken.

Die Kristalle werden abfiltriert und der Filterkuchen zur Verdrängung der Schwefelsäure-Mutterlauge zweckmäßig mit frischer, vorzugsweise 20 bis 30 %iger Schwefelsäure gewaschen und gründlich abgesaugt. Man erhält einen Filterkuchen mit einer geringen Restfeuchte an wässriger Schwefelsäure, jedoch frei oder jedenfalls praktisch frei von organischen Nebenprodukten. Falls auch ein von Restschwefelsäure freies Produkt gewünscht wird, kann man die Kristalle noch umkristallisieren.

Falls jedoch nicht die Gewinnung von reinem 6-Methyl-3,4-dihydro-1,2,3-oxathiazin-4-on-2,2-dioxid, sondern der Erhalt von dessen nicht-toxischen Salzen — insbesondere des Kaliumsalzes — beabsichtigt ist, können die noch eine geringe Restfeuchte an wässriger Schwefelsäure enthaltenden Kristalle des 6-Methyl-3,4-dihydro-1,2,3-oxathiazin-4-on-2,2-dioxids durch Neutralisation gleich in die entsprechenden Salze überführt werden. Die Neutralisation geschieht im Prinzip in der gleichen Weise wie dies in der EP-A-0155634 beschrieben ist. Zu diesem Zweck wird das zu neutralisierende Produkt in Wasser oder in organischen Lösemitteln wie z. B. Alkoholen, Ketonen, Estern oder Ethern — vorzugsweise nur in Wasser — gelöst und mit einer entsprechenden Base, insbesondere einer Kaliumbase wie z. B. KOH, $KHCO_3$, $K_2CO_3$ oder einem K-Alkoholat, neutralisiert.

In einer bevorzugten Ausführungsform der Neutralisation wird das 6-Methyl-3,4-dihydro-1,2,3-oxathiazin-4-on-2,2-dioxid in der etwa gleichen Menge Wasser bei Temperaturenzwischen etwa 0 und 100 °C, vorzugsweise zwischen etwa 20 und 80 °C, gelöst und mit etwa 45 bis 50 %iger Kalilauge neutralisiert. Nach der Neutralisation wird die wässrige Suspension des Kaliumsalzes abgekühlt und filtriert. Wegen der relativ guten Löslichkeit des Kaliumsalzes in Wasser ist eine Abkühlung auf 0 bis etwa 10 °C empfehlenswert. Da das nicht umkristallisierte 6-Methyl-3,4-dihydro-1,2,3-oxathiazin-4-on-2,2-dioxid noch eine geringe Restfeuchte an Schwefelsäure enthält, entsteht bei der Neutralisation mit einer Kaliumbase neben dem gewünschten Kaliumsalz auch eine geringe Menge an Kaliumsulfat. Aufgrund der guten Löslichkeit des Kaliumsulfats in Wasser verbleibt aber praktisch das gesamte Kaliumsulfat in der Neutralisationsmutterlauge.

Das Kaliumsalz des 6-Methyl-3,4-dihydro-1,2,3-oxathiazin-4-on-2,2-dioxids (Acesulfam K) hat nach der Trocknung eine Reinheit von über 99,5 %. Der geringe Rest besteht praktisch ausschließlich aus Kaliumsulfat.

Für diese Ausführungsform der Neutralisation beträgt der Isolierungsgrad an Acesulfam K etwa 80-90 % d. Th., bezogen auf das eingesetzte 6-Methyl-3,4-dihydro-1,2,3-oxathiazin-4-on-2,2-dioxid. Der in der

Neutralisationsmutterlauge verbleibende Anteil von Acesulfam K entspricht dessen Löslichkeit in Wasser. Der Isolierungsgrad kann erhöht werden, indem weniger Wasser zum Lösen des 6-Methyl-3,4-dihydro-1,2,3-oxathiazin-4-on-2,2-dioxids verwendet wird ; jedoch wird dann die erhaltene Acesulfam K-Suspension dicker.

Wenn ein noch geringerer Gehalt an Kaliumsulfat im Acesulfam K gewünscht wird, so kann noch eine Umkristallisation aus Wasser angeschlossen werden. Das Acesulfam K wird so in praktisch 100 %iger Reinheit erhalten. Die Mutterlauge der Umkristallisation kann zum Lösen des 6-Methyl-3,4-dihydro-1,2,3-oxathiazin-4-on-2,2-dioxids zurückgeführt werden, so daß kein Ausbeuteverlust entsteht.

Eine weitere Ausbeuteoptimierung kann gegebenenfalls auch noch dadurch erfolgen, daß das nach der Abfiltration des 6-Methyl-3,4-dihydro-1,2,3-oxathiazin-4-on-2,2-dioxids aus der Schwefelsäurephase in der Schwefelsäuremutterlauge gelöst verbleibende Produkt mit einem mit Wasser nicht mischbaren inerten organischen Lösemittel — möglichst dem gleichen, welches auch für die Durchführung der Ringschlußreaktion verwendet wurde — extrahiert und mit dem Reaktionsaustrag vor der Destillation des Lösemittels vereint wird.

Auch das Einengen der Neutralisationsmutterlauge kann als Maßnahme der weiteren Ausbeuteoptimierung in Betracht gezogen werden.

Die Ausbeuten des erfindungsgemäßen Ringschlußverfahrens mit anschließender Produktgewinnung liegen in der Größenordnung der in den drei vorerwähnten Patentanmeldungen angegebenen Ausbeuten ; durch die Erfindung wird jedoch eine höhere Produktreinheit erhalten.

Die erfindungsgemäß im Anschluß an die Ringschlußreaktion durchgeführte Produktgewinnung kann schließlich auch zur Reinigung von anderweitig hergestelltem unreinem 6-Methyl-3,4-dihydro-1,2,3-oxathiazin-4-on-2,2-dioxid benutzt werden.

Erfindungsgegenstand ist daher auch ein Verfahren zur Reinigung von 6-Methyl-3,4-dihydro-1,2,3-oxathiazin-4-on-2,2-dioxid, das dadurch gekennzeichnet ist, daß man rohes 6-Methyl-3,4-dihydro-1,2,3-oxathiazin-4-on-2,2-dioxid aus wässriger Schwefelsäure — vorzugsweise etwa 20 bis 90 %iger, insbesondere etwa 50 bis 85 %iger Schwefelsäure — umkristallisiert.

Im rohen 6-Methyl-3,4-dihydro-1,2,3-oxathiazin-4-on-2,2-dioxid vorhandene etwaige anorganische und organische Verunreinigungen werden durch diese Umkristallisation vollständig oder jedenfalls praktisch vollständig entfernt.

Die folgenden Beispiele sollen der weiteren Erläuterung der Erfindung dienen. Nach den Erfindungsbeispielen (A) folgt noch ein Vergleichsbeispiel (B), aus welchem hervorgeht, daß ein weniger reines 6-Methyl-3,4-dihydro-1,2,3-oxathiazin-4-on-2,2-dioxid erhalten wird, wenn man dessen Gewinnung nicht aus der Schwefelsäurephase, sondern aus der organischen Phase vornimmt. In den Beispielen wird 6-Methyl-3,4-dihydro-1,2,3-oxathiazin-4-on-2,2-dioxid mit « ASH », dessen Kaliumsalz mit « ASK » abgekürzt.

Herstellung des für die Beispiele verwendeten Ausgangs-Acetoacetamid-N-sulfonats

In 250 ml $CH_2Cl_2$ wurden 48,6 g (= 0,5 Mol) Amidosulfonsäure unter Kühlen mit 52,5 g (= 0,53 Mol) Triethylamin so neutralisiert, daß eine Temperatur von + 30 °C nicht überschritten wurde. Es wurden 3 g Essigsäure zugegeben. 46,3 g (= 0,55 Mol) Diketen wurden bei 0 °C in 60 min. zugetropft. Anschließend wurde 60 min. bei 0 °C und 6 Stunden bei Raumtemperatur gerührt.

A) Erfindungsbeispiele

Beispiel 1

a) Ringschluß und Hydrolyse :

500 ml $CH_2Cl_2$ wurden in einem Reaktionsgefäß vorgelegt und auf — 30 °C gekühlt. Die wie vorstehend beschrieben hergestellte Lösung des Triethylammonium-acetoacetamid-N-sulfonats und 120 ml (= 2,8 Mol) $SO_3$ wurden gleichzeitig eingetropft. Die Temperatur im Reaktionsgefäß überstieg — 30 °C nicht. Anschließend wurde weitere 30 min. bei — 30 °C gerührt. Dann wurden 162 ml Wasser bei — 15 °C bis — 10 °C zugetropft und 1,5 Stunden bei 0 °C gerührt.

Der Reaktionsaustrag war ein dreiphasiges Gemisch aus einer $CH_2Cl_2$-Phase, einer $H_2SO_4$-Phase und festem ASH.

b) Aufarbeitung :

Aus dem Reaktionsaustrag wurde das $CH_2Cl_2$ in einer Vakuumdestillation bei 500 mbar und 24 °C Siedetemperatur abdestilliert. Dabei wurde im Sumpf eine maximale Temperatur von 40 °C nicht überschritten. Die Suspension aus ASH in Schwefelsäure wurde auf 0 °C gekühlt und mit einer Glasfilternutsche abfiltriert. Der Filterkuchen wurde auf der Nutsche mit 50 ml 30 %iger $H_2SO_4$ gewaschen und filtriert.

Durch potentiometrische Titration wurde der ASH-Filterkuchen analysiert. Er enthielt 92 % ASH und 2,5 % $H_2SO_4$. Der Rest zu 100 % war Wasser. In der HPLC (= Hochdruckflüssigchromatographie)-Analyse konnten keine organischen Nebenprodukte festgestellt werden.

Die Ausbeute an ASH betrug 49,8 g (= 0,31 Mol) = 61 % d. Th., bezogen auf die ursprünglich eingesetzte Amidosulfonsäure.

Zwecks Überführung in das K-Salz wurde das ASH noch in 61 ml Wasser bei 30 °C gelöst und bei 30 °C mit 50 %iger KOH bis pH = 7 neutralisiert. Die Suspension des entstandenen ASK wurde auf 0 °C gekühlt und filtriert. Nach Trocknen im Vakuumtrockenschrank bei 267 mbar und 60 °C wurden 52,3 g ASK erhalten, entsprechend einer Ausbeute von 52 % d. Th., bezogen auf die Amidosulfonsäure. Das ASK war weiß. Die Reinheit wurde mittels potentiometrischer Titration und HPLC bestimmt.

ASK : 99,9 %. $K_2SO_4$ : 0,1 % ; KCl : 20 ppm. Organische Nebenprodukte konnten mit der HPLC nicht nachgewiesen werden.

Nach Umkristallisation aus der 0,8-fachen Menge Wasser betrug der Gehalt an $K_2SO_4$ im ASK unter 100 ppm.

## Beispiel 2

Aus dem nach Beispiel 1a) hergestellten Reaktionsaustrag wurde das $CH_2Cl_2$ bei Normaldruck, Kp/lbar = 42 °C, abdestilliert. Gegen Ende der Destillation wurde eine maximale Sumpftemperatur von 70 °C nicht überschritten. Beim Abkühlen schieden sich aus der Schwefelsäurephase Kristalle ab, die bei 0 °C abfiltriert und mit 50 ml 30 %iger Schwefelsäure gewaschen wurden. Der Filterkuchen enthielt 90 % ASH und 3,5 % $H_2SO_4$.

Die Ausbeute betrug 54 g (= 0,33 Mol) = 66 % d. Th., bezogen auf Amidosulfonsäure.

Die Neutralisation wurde wie in Beispiel 1b) beschrieben durchgeführt. Es wurde ein ASK erhalten mit einem Gehalt von 99,8 % ASK und 0,2 % $K_2SO_4$.

Die Ausbeute betrug 54 g (= 0,27 Mol) = 54 % d. Th., bezogen auf Amidosulfonsäure.

Nach einer Umkristallisation betrug der Gehalt an ASK 100 %. In der HPLC-Analyse wurden keine Nebenprodukte festgestellt.

## Beispiele 3-6

Der nach Beispiel 1a) hergestellte Reaktionsaustrag wurde wie in Beispiel 2 beschrieben aufgearbeitet und neutralisiert. Nach dem Abfiltrieren des ASH aus der Schwefelsäurephase und dem Waschen des ASH mit 30 %iger Schwefelsäure wurde die Schwefelsäurephase zweimal mit 250 ml Methylenchlorid extrahiert. Der Extrakt wurde mit dem Reaktionsaustrag des jeweils folgenden Versuchs vereint. Die bei der Umkristallisation des ASK anfallende Mutterlauge wurde zum Lösen des ASH bei der Neutralisation des jeweils folgenden Versuchs eingesetzt. Die Ergebnisse der Beispiele 3-6 können der Tabelle 1 entnommen werden.

(Siehe Tabelle Seite 12 ff.)

## Beispiel 7

Der nach Beispiel 1a) hergestellte Reaktionsaustrag wurde aus einem gerührten Einsatzgefäß auf einen Dünnschichtverdampfer dosiert. Durchsatzmenge und Heizleistung wurden so eingestellt, daß sich eine Ablauftemperatur von 60 °C ergab. Aus der ablaufenden Schwefelsäurephase kristallisiert beim Abkühlen das ASH aus. Einschließlich der Extraktion von ASH aus der Schwefelsäuremutterlauge und der Rückführung der Kristallisationsmutterlauge wurde eine ASH-Ausbeute von 71 % d. Th. erhalten. Der ASH-Filterkuchen enthielt 94 % ASH und 2 % $H_2SO_4$.

Die Neutralisation wurde entsprechend Beispiel 1 ausgeführt. Das erhaltene ASK hatte einen Gehalt von 99,6 % ASK und 0,3 % $K_2SO_4$.

## B) Vergleichsbeispiel

Bei dem nach Erfindungsbeispiel (A)1 hergestellten Reaktionsaustrag wurde die Methylenchloridphase von der Schwefelsäurephase abgetrennt und die Schwefelsäurephase zweimal mit 250 ml $CH_2Cl_2$ ausgeschüttelt. Die vereinigten Methylenchloridphasen wurden mit Natriumsulfat getrocknet. Nach dem Abdestillieren des $CH_2Cl_2$ wurde das ASH als ein hellgelber öliger Rückstand erhalten.

Nach lösen in 60 ml $H_2O$ wurde das ASH mit 50 %iger KOH zum ASK neutralisiert und im Vakuum getrocknet. Man erhielt ein hellgelbes ASK mit einem Gehalt von 85 % ASK und 5 % Kaliumsulfat.

Die Ausbeute betrug 66 g (= 0,33 Mol) = 65 % d. Th., bezogen auf Amidosulfonsäure.

Tabelle 1

| Beispiel | | ASH-Filterkuchen | | ASK | | | ASK umkristallisiert | | |
|---|---|---|---|---|---|---|---|---|---|
| | | Ausbeute | Gehalt | Ausbeute | Reinheit i. % | | Ausbeute | Reinheit i. % | |
| | | % | % | % | ASK | $K_2SO_4$ | % | ASK | $K_2SO_4$ |
| 3 | Startversuch | 69 | 91 | 56 | 99,5 | 0,2 | 48 | 100 | 0,03 |
| 4 | Rückführung von Extrakt.u.Mutterl. | 67 | 90 | 63 | 99,8 | 0,1 | 54 | 100 | 0,03 |
| 5 | " | 71 | 91 | 67 | 99,8 | 0,2 | 57 | 100 | 0,01 |
| 6 | " | 71 | 93 | 66 | 99,5 | 0.3 | 56 | 100 | 0,03 |

**Patentansprüche**

1. Verfahren zur Herstellung von 6-Methyl-3,4-dihydro-1,2,3-oxathiazin-4-on-2,2-dioxid durch Ringschluß eines Acetoacetamid-Derivats, dadurch gekennzeichnet, daß man als Acetoacetamid-Derivat Acetoacetamid-N-sulfonsäure oder deren Salze — gelöst in einem mit Wasser nicht mischbaren inerten organischen Lösemittel — verwendet, daß man den Ringschluß durch die Einwirkung der mindestens äquimolaren Menge $SO_3$ — gegebenenfalls in gleicher Weise gelöst in einem mit Wasser nicht mischbaren inerten organischen Lösemittel oder auch in einem inerten anorganischen Lösemittel — durchführt, daß man im Falle des Einsatzes einer äquimolaren Menge $SO_3$ nach beendeter Ringschlußreaktion 20 bis 90 %ige wässrige Schwefelsäure zusetzt bzw. — im Falle des Einsatzes einer mehr als äquimolaren Menge $SO_3$ — das nach der Ringschlußreaktion als $SO_3$-Addukt anfallende 6-Methyl-3,4-dihydro-1,2,3-oxathiazin-4-on-2,2-dioxid hydrolysiert, daß man aus dem resultierenden Mehrphasengemisch das inerte organische Lösemittel abdestilliert, und aus der verbleibenden wässrig-schwefelsauren Phase das 6-Methyl-3,4-dihydro-1,2,3-oxathiazin-4-on-2,2-dioxid durch Kristallisation gewinnt.

2. Verfahren nach Anspruch 1, dadurch gekennzeichnet, daß man Lösungen der Acetoacetamid-N-sulfonsäure oder von deren Salzen und von $SO_3$ in dem gleichen mit Wasser nicht mischbaren inerten organischen Lösemittel verwendet.

3. Verfahren nach Anspruch 1 oder 2, dadurch gekennzeichnet, daß man als organisches Lösungsmittel einen aliphatischen Chlorkohlenwasserstoff verwendet.

4. Verfahren nach einem oder mehreren der Ansprüche 1 bis 3, dadurch gekennzeichnet, daß man als organisches Lösungsmittel Methylenchlorid verwendet.

5. Verfahren nach einem oder mehreren der Ansprüche 1 bis 4, dadurch gekennzeichnet, daß man den Ringschluß durch die Einwirkung einer mehr als äquimolaren Menge $SO_3$, in Bezug auf Acetoacetamid-N-sulfonsäure oder von deren Salzen, durchführt.

6. Verfahren zur Reinigung von 6-Methyl-3,4-dihydro-1,2,3-oxathiazin-4-on-2,2-dioxid, das nach dem Verfahren nach einem oder mehreren der Ansprüche 1 bis 5 erhältlich ist, dadurch gekennzeichnet, daß man rohes 6-Methyl-3,4-dihydro-1,2,3-oxathiazin-4-on-2,2-dioxid aus 20 bis 90 %iger wässriger Schwefelsäure umkristallisiert.

7. Verfahren nach Anspruch 6, dadurch gekennzeichnet, daß man für die Umkristallisation 50 bis 85 %ige Schwefelsäure verwendet.

**Claims**

1. A process for the preparation of 6-methyl-3,4-dihydro-1,2,3-oxathiazin-4-one-2,2-dioxide by cyclizing an acetoacetamide derivative, which comprises using acetoacetamide-N-sulfonic acid or its salts — dissolved in a water-immiscible, inert, organic solvent — as the acetoacetamide derivative, carrying out the cyclization by treatment with an at least equimolar amount of $SO_3$ — if appropriate similarly dissolved in a water-immiscible, inert, organic solvent or in an inert, inorganic solvent — in the event that an equimolar amount of $SO_3$ is employed, adding aqueous sulfuric acid of 20 to 90 % strength when the cyclization reaction is complete, or — in the event that the amount of $SO_3$ employed is more than equimolar — hydrolyzing the 6-methyl-3,4-dihydro-1,2,3-oxathiazin-4-one-2,2-dioxide obtained in the form of the $SO_3$-adduct after the cyclization reaction, removing the inert, organic solvent from the resulting multi-phase mixture by distillation, and isolating the 6-methyl-3,4-dihydro-1,2,3-oxathiazin-4-one-2,2-dioxide by cristallization from the remaining aqueous sulfuric acid phase.

2. The process as claimed in claim 1, wherein solutions of acetoacetamide-N-sulfonic acid or salts thereof and of $SO_3$ in the same water-immiscible, inert, organic solvent are used.

3. The process as claimed in claims 1 or 2, wherein an aliphatic chlorinated hydrocarbon is used as the organic solvent.

4. The process as claimed in one or more of claims 1 to 3, wherein methylene chloride is used as the organic solvent.

5. The process as claimed in one or more of claims 1 to 4, which comprises carrying out the cyclization by treatment with a greater than equimolar amount of $SO_3$ which is more than equimolar, relative to acetoacetamide-N-sulfonic acid or salts thereof.

6. A process for the purification of 6-methyl-3,4-dihydro-1,2,3-oxathiazin-4-one-2,2-dioxide, which can be obtained by the process as claimed in one or more of claims 1 to 5, which comprises recrystallizing crude 6-methyl-3,4-dihydro-1,2,3-oxathiazin-4-one-2,2-dioxide from aqueous sulfuric acid of 20 to 90 % strength.

7. The process as claimed in claim 6, wherein sulfuric acid of 50 to 85 % strength is used for the recrystallization.

**Revendications**

1. Procédé pour préparer le méthyl-6 dihydro-3,4 oxathiazine-1,2,3 one-4 dioxyde-2,2 par cyclisation

d'un dérivé de l'acétylacétamide, procédé caractérisé en ce que on utilise comme dérivé de l'acétylacétamide l'acide acétylacétamide-N-sulfonique ou un sel de celui-ci, en solution dans un solvant organique inerte non miscible à l'eau, on effectue la cyclisation en faisant agir une quantité au moins équimolaire de $SO_3$, éventuellement dissous de la même façon dans un solvant organique inerte non miscible à l'eau ou encore dans un solvant inorganique inerte, dans le cas où l'on a utilisé une quantité équimolaire de $SO_3$ on ajoute, après la fin de la réaction de cyclisation, un acide sulfurique aqueux d'une concentration comprise entre 20 et 90 %, ou, dans le cas où l'on a utilisé une quantité de $SO_3$ supérieure à la quantité équimolaire, on hydrolyse le méthyl-6 dihydro-3,4 oxathiazine-1,2,3 one-4 dioxyde-2,2 obtenu sous la forme d'un produit d'addition de $SO_3$ (« $SO_3$-adduit ») après la réaction de cyclisation, du mélange à plusieurs phases obtenu on chasse par distillation le solvant organique inerte, et, à partir de la phase aqueuse sulfurique qui reste, on isole le méthyl-6 dihydro-3,4 oxathiazine-1,2,3 one-4 dioxyde-2,2 par cristallisation.

2. Procédé selon la revendication 1, caractérisé en ce qu'on utilise des solutions de l'acide acétylacétamide-N-sulfonique, ou d'un de ses sels, et de $SO_3$ dans le même solvant organique inerte non miscible à l'eau.

3. Procédé selon l'une des revendications 1 et 2, caractérisé en ce qu'on utilise, comme solvant organique, un hydrocarbure aliphatique chloré.

4. Procédé selon l'une quelconque des revendications 1 à 3, caractérisé en ce qu'on utilise le chlorure de méthylène comme solvant organique.

5. Procédé selon l'une quelconque des revendications 1 à 4, caractérisé en ce qu'on effectue la cyclisation en faisant agir une quantité de $SO_3$ supérieure à la quantité équimolaire par rapport à l'acide acétyl-acétamide-N-sulfonique ou à ses sels.

6. Procédé pour purifier le méthyl-6 dihydro-3,4 oxathiazine-1,2,3 one-4 dioxyde-2,2 qui peut être obtenu selon l'une quelconque des revendications 1 à 5, procédé caractérisé en ce qu'on recristallise le méthyl-6 dihydro-3,4 oxathiazine-1,2,3 one-4 dioxyde-2,2, qui peut être obtenu selon l'une quelconque des revendications 1 à 5, procédé caractérisé en ce qu'on recristallise le méthyl-6 dihydro-3,4 oxathiazine-1,2,3 one-4 dioxyde-2,2 brut dans un acide sulfurique aqueux d'une concentration comprise entre 20 et 90 %.

7. Procédé selon la revendication 6 caractérisé en ce qu'on utilise, pour la recristallisation, un acide sulfurique d'une concentration comprise entre 50 et 85 %.